# EUROPEAN PATENT APPLICATION

(11) **EP 1 754 972 A1**
(43) Date of publication of application: **21.02.2007**
(21) Application number: 05107562.0
(22) Date of filing: 17.08.2005
(51) Int. Cl.: G01N 33/58, G01N 33/68

(54) **Folded peptides containing metal binding pockets for protein tagging**

(71) Applicant: Agilent Technologies, Inc., Santa Clara, CA 95051 (US)
(72) Inventor: Grimm, Rudolf, 82131 Gauting (DE)
(74) Representative: Barth, Daniel Mathias

(57) **Abstract**

A folded peptide is provided, wherein the folding of the peptide provides at least one pocket being adapted for the uptake of a metal ion, thus leading to a labeling of said peptide. Furthermore, a plurality of peptides is provided, wherein each single peptide of said plurality of peptides is differentiable from the other peptides due to labeling with metal ions. A method for preparing said labeled peptides is provided.
These peptides are adapted for tagging on proteins and are therefore useful in proteomics. The preferred metal ions are from the lanthanide series, e.g. En, Ce, Nd, Yb, Sm, Gd, Er.

## Description

The present invention relates to an improved tagging of proteins.

### BACKGROUND ART

A number of different technologies has been developed aiming for an improved proteomics analysis. Proteomics is the qualitative and quantitative analysis of proteins in cells or organisms, allowing the researcher to conclude about the state of a disease such as cancer e. g., or about the development of stress and other symptoms being based on protein activities in biomaterials such as cells.

In order to study the development of the mechanisms proceeding in an organism one may take samples of the cells or tissues of interest and subject them to analysis. A number of sample preparation steps has to be carried out prior to tracking the proteins of interest: Those proteins have to be analyzed, identified and separated from a mixture of proteins.

Multiple technologies have been developed in order to carry out the mentioned separating, analyzing and identification steps. Identification may be performed by mass spectrometry (MS), e.g, requiring the analysis of isolated proteins or peptide fragments before mapping or tandem MS gives the desired sequence information. To facilitate the differentiation and allow the assignment of the resulting spectra to single proteins one may tag the proteins. The tagging can be performed in different ways, the efficiency of the proceeding and the reliability of the results being based on the selection of the tagging method.

One of said tagging methods known in the art is the tagging of proteins with reagents having different masses, in particular basing on the mass difference of the isotopes hydrogen and deuterium, known as "Isotope-Coded Affinity Tags" (ICAT). ICAT is limited to differential analysis of two samples due to the use of two different masses. (Ranish et al. Nature Genet. 33, 2003, 349-355; Zhou et al., Nature Biotechnol. 19, 2002, 512-515.)

Another protein labeling technique referred to as "ECAT" is based on tagging with different element-coded metal chelates affording affinity chromatography, quantification and identification of a tagged peptide from a complex mixture. (Whetstone et al., Bioconjug. Chem., 2004 Jan-Feb; 15 (1): 3-6.)

Labeling of proteins is described under http://www.appliedbiosystems.com, 2004, too. Herein, all peptides can be labeled simultaneously on up to four different biological samples by use of amine-specific, stable isotope reagents.

Furthermore, the labeling of bovine serum albumin (BSA) has been described. Performing of the labeling is done with a europium chelator, which is a rare earth bifunctional chelate. (Guang Pu Xue Yu Guang Puu Fen Xi, 2004 Jul;24 (7), 795-8.)

Peptide-based metal ion-labeled compositions for use as pharmaceuticals and methods of labeling peptides have been described in U.S. 5,690,905 to Zamora et al., who additionally depict the labeling of peptides with metal ions in U.S. 5,861,139.

U.S. 2002/0076739 A1 to R. Aebersold et al. gives another description of a methodology for the rapid quantitative analysis of proteins or protein functions in complex mixtures. Herein, the authors refer to affinity labeled proteins or protein functions in mixtures of proteins comprising three portions: An affinity label, which is covalently linked to a protein reactive group through a linker group, wherein the linker group may comprise an isotopic label.

### DISCLOSURE

It is an object of the invention to provide an improved tagging of peptides and proteins. The object is solved by the independent claims. Preferred embodiments are shown by the dependent claims.

According to embodiments of the present invention, an improved tagging of peptides is provided, comprising the labeling of proteins and peptides with lanthanoid elements.

A preferred embodiment of the present invention comprises a peptide that is folded in a manner that a three-dimensional cage or pocket is provided in which a metal ion can be inserted. Due to the binding of the metal ion to the peptide labeling of said peptide is provided.

Embodiments of the invention refer to lanthanoid elements as the metal ions to be inserted in said pockets since a number of them occurs naturally in isotopic forms and, hence, offers a plurality of labeling options.

Further embodiments of the invention refer to a plurality of peptides which are labeled as described in the forgoing embodiments and which may now be tagged on different proteins resulting from one original sample in order to allow differentiation of said multiplexed proteins in analytical procedures.

After all a method is provided to prepare peptides according to embodiments of the present invention, wherein said method teaches labeling of peptides with metal ions prior to tagging them on proteins of interest. Said tagging may require the insertion of linking and probably additional spacing elements.

### BRIEF DESCRIPTION OF DRAWINGS

Other objects and many of the attendant advantages of embodiments of the present invention will be readily appreciated and become better understood by reference to the following more detailed description of preferred embodiments in connection with the accompanied figures.

Fig. 1 shows a table listing the lanthanoid elements and their naturally occurring isotopes,

Fig. 2 shows some lanthanoid elements exemplarily, each of which connected to a reactive end group of a protein via a linker.

Fig. 3 shows some lanthanoid elements complexed with the specific cage peptide exemplarily, each of which connected to a reactive end group of a protein through a linker and via a spacer,

Fig. 4 shows exemplarily a number of isotopes of the lanthanoid Ytterbium complexed with their specific cage peptides, each of them being attached to a reactive end group of another protein through a linker and via a spacer.

In the description and in the claims which follow, reference will be made to the following terms which shall be defined to have the herewith explained meanings:

A "linker" is meant to be understood as a molecule which allows stable bonding of the target molecule to the reactive site of the labelled peptide.

Herein, a "spacer" is defined as a molecule which is located in-between the target molecule and the labelled peptide in order to prevent steric hindrance between the both.

A "pocket" is a three-dimensional molecule peptide structure which is suitable to envelope or encase a object such as a metal-ion, whereby a bonding or "fitting" is provided between metal ion and pocket comparable to a key / key seat bonding.

Generally, the multiplexed assaying of proteins or enzymes is difficult to be performed and the techniques which are presently available for analytical multiplexed assays may not be sufficient with respect of their efficiency. After all, it still seems problematic to analyze a plurality of samples in parallel. Accordingly, the requirement of assaying a plurality of protein samples resulting from one origin sample which has been multiplexed is still a challenge. It is most advantageous to perform parallel assaying of multiplexed samples since this allows subjecting of a "set" of samples under controllable and consistent or constant conditions and constant ambient parameters, whereby the reliability on the results is increased. Furthermore, the performance of kinetic studies requires subjecting a plurality of samples having an identical origin to an identical process, too.

Accordingly, the different samples need to be distinguishable, which may be achieved by tagging the molecule of interest or "target molecules", respectively, to labeled molecules.

The tagging can be obtained according to an embodiment of the present invention: Said embodiment refers to a peptide, whereby the peptide is as small as can be, and which peptide can be a chemically synthesized or a natural peptide. It provides a pocket due to folding. The folding and formation of the pocket may be achieved by performing overexpressing. One may wish to genetically perform said folding-procedure by utilizing a transgenic organism such as E. coli, or one may use yeast or other appropriate organism as vehicle to carry out the procedure of overexpressing. If necessary, the peptide is subjected to a purification procedure after exiting said transgenic organism. Generally, the process of overexpressing is known to the one skilled in the art.

The pocket which is generated is adapted to be loaded with a metal ion in order to achieve labeling of the peptide. Advantageously, one may chose lanthanoid elements for insertion into the pocket, since most of the lanthanoid metals are naturally polyisotopic.

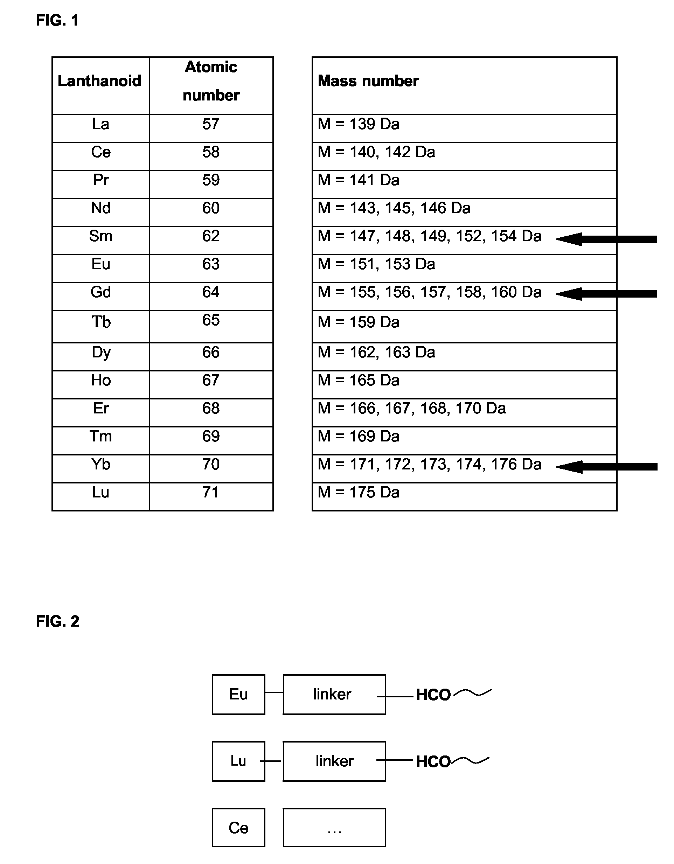

**Figure 1** gives the lanthanoid elements as they are listed in the periodic system. Their atomic numbers reach from 57 to 71 and the mass numbers of the isotopes occurring naturally in noteworthy percentages reach from approximately 138 to 196 Dalton.

Ytterbium (Yb), for example, has a number of five naturally occurring isotopes and, hence, a number of five distinguishable peptides can be generated by loading said pockets of five peptides having an identical molecule structure with said five different isotopes of one single lanthanoid element. Selecting Samarium (Sm) and Gadolinium (Gd) have five isotopic forms, too. Accordingly, one could prepare a number of 15 different and thus distinguishable peptides by selection of only three lanthanoid elements. Each of which peptides is adapted to be tagged on 15 identical target molecules, making them distinguishable.

Furthermore, one may wish to increase the number of distinguishable proteins: This could be achieved by crosslinking two ore more different isotope binding peptides with one protein, for example. Crosslinking of each of the Ytterbium isotopes with the identical and four different isotopes would lead to 15 different combinations and, hence, to 15 distinguishable protein samples; crosslinking of more than two isotopes would increase the number of distinguishable protein samples again.

Furthermore, the choice of lanthanoides may be advantageous according to another aspect: They show a great sensitivity in ICP-MS analysis and could therefore provide an optimized reliability in analytical results. Also ICP-MS allows the analysis of proteins over a concentration range of 9 orders of magnitude which is unsurpassed by any other mass spectrometry method.

It has to be taken into consideration that due to the relative atom masses the size of two different lanthanoid elements may differ remarkably: Whereas a Lanthan (La) atom has a mass number of about 138 Dalton, one of the Tamarium isotopes (Tm) has abut 168 Dalton - accordingly the Tamarium isotope requires a much bigger pocket for insertion than the Lanthan isotope does. Hence, the designing of said peptide pockets has to performed carefully and with respect to the specific metal ions that are intended to be bound in said pockets. Taking into consideration that the metal ion has to be bound tightly in its pocket, the size of the pocket needs to be custom-tailored, allowing an exact fit of the metal ions in their housing, thereby not allowing the metal ion to have free play.

Once being labeled and thus being adapted to function as a tag, the peptide may be tagged on a target molecule, which advantageously can be a protein. The target molecule might be a recombinant protein such as avidin, e.g, or an antibody, only to give some examples.

The tagging may be performed directly, maybe genetically, but in a preferred embodiment a linker is located in-between the labeled peptide and the protein, as indicated in **figure 2**. A suitable linker allows complete reaction between a target molecule and the reactive site of the peptide tag to form a stable chemical bond. A chemical linker should have at least one reactive group providing the linkage, said reactive group being one of -NCS, -CHO, or -SH for example. So, said linker could be provided by an aldehyde group which could react with the amino groups of lysine residues of the protein of interest, or with the amino-terminal amino group. Alternatively, it can consist of a thiol group which can react with the thiol group of cysteine residues of the target protein or peptide. These examples are intended to be given only exemplarily.

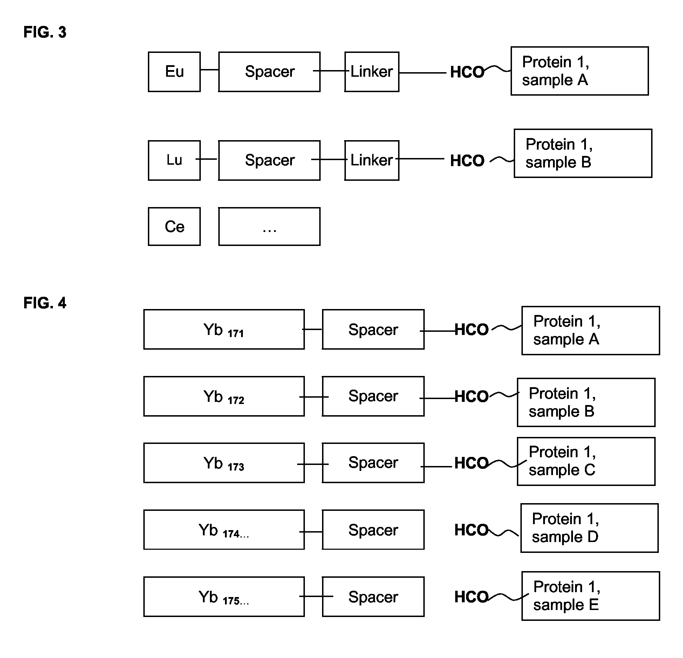

Furthermore, it is advantageous to provide a spacer which should be located whether directly or indirectly between the labeled peptide and the target molecule. The placement of the spacer is schematically shown in **figure 3**. "Indirectly" herein means, that the spacer could be located adjacent to the linker on the one hand side, whereas the target molecule or alternatively the protein is arranged the other side. Said spacer is adapted to prevent steric hindrance which may be a result of the sizes and loadings of said labeled peptide and said target molecule. The spacer might be a carbon chain such as a C-6 carbon chain or a small molecule such as biotin. Other molecules suitable to prevent steric hinderance may be used alternatively.

The labeled peptide may be tagged through said linker genetically to said target molecule, too.

**Figure 4** gives an idea, how a number of five samples which stem from one protein "Protein 1", could be tagged on five different peptides which are distinguishable due to labeling with five Ytterbium isotopes. So, a plurality of distinguishable peptides can be obtained by labeling whether with:
- different metal ions, in particular with different lanthanoid elements,
- or with different isotopes of one single lanthanoid element, as shown in **fig. 4.**

After all, the preparation of said labeled peptides generally comprises the steps of overexpressing a small peptide. When the pocket is designed utilizing said overexpression, the pocket becomes loaded with a metal ion such as a lanthanoid element. Advantageously, the peptides are loaded when being in solution. Accordingly, the loading procedure requires conditions such as an optimal solubility of said metals in the peptide containing solution. One may advantageously wish to perform the process in a buffered solution; a buffer such as TRIS or as a phosphate buffer could be used, providing a pH of about 7,4; furthermore salt components can be added. The loading of the pockets with the metal ions may be performed during the step of overexpressing or afterwards. Prior to loading, a chemically synthesized peptide should be subjected to purification.

The next step would be then the tagging a target molecule such as a protein with at least one of said labeled peptides. As described above, one may tag the target molecule on more than one labeled protein in order to distinguish a maximum number of protein samples. The tagging is performed after a linking molecule has been coupled to the labeled peptide and, optionally, after a spacer has been introduced between two of the labeled peptide, the linker and the target molecule.

## Claims

1. A folded peptide, wherein the folding of the peptide provides at least one pocket, which pocket is adapted for the uptake of a metal ion whereby a labeled peptide is provided.

2. The peptide of claim 1, comprising at least one of the features:
• the peptide is a single peptide of minimum size;
• the folded peptide can be a chemically synthesized peptide or a natural peptide;
• said pocket has a size that is designed to envelope said lanthanoid element free from play.

3. The peptide of claim 1 or any one of the above claims, wherein the metal ion which is provided to be taken up is one of the lanthanoid elements having the atomic numbers 57 to 71.

4. The peptide of the preceding claim 3, which lanthanoid elements comprise the natural and artificial isotopes of Europium, Cer, Neodymium, Ytterbium, Samarium, Gadolinium and Erbium.

5. The peptide according to claim 1 or any one of the preceding claims, wherein at least one of said labeled peptides is tagged on a target molecule, said target molecule being a protein or a peptide.

6. The peptide of the preceding claim, comprising at least one of the features:
• said labeled peptide is connected to a linker, said linker being adapted to provide linkage between said labeled peptide and said target molecule;
• the linker is a chemical linker having at least one reactive group providing the linkage, said reactive group being one of -NCS, -CHO, or ―SH;
• a spacer is located between the labeled peptide and one of the linker or the target molecule, said spacer being adapted prevent steric hindrance due to interactions of said labeled peptide and said target molecule;
• the labeled peptide is tagged through said linker genetically to said target molecule;
• the target molecule is a recombinant protein such as avidin or antibodies.

7. A plurality of peptides, in particular peptides according to claim 1 or any one of the above claims, wherein each single peptide of said plurality of peptides is analytically distinguishable from the other peptides due to labeling with:
- different metal ions, in particular different lanthanoid elements,
- different isotopes of one single metal ion, in particular different isotopes of one single lanthanoid element.

8. The plurality of peptides according the preceding claim, comprising at least one of the features:
• each of said distinguishable peptides is tagged to a different protein comprised in a mixture;
• said different proteins in said mixture result from multiplexing of an origin protein sample.

9. A method for preparing a folded peptide, comprising at least one of the features:
- overexpressing a peptide,
- providing a pocket due to said overexpression,
- loading said pocket with a metal ion,
whereby the loading may be performed during the step of overexpressing or afterwards, accordingly providing a labeled peptide.

10. The method of the above claim, comprising at least one of the features:
• purifying said peptide prior to loading said pocket with said metal ion;
• tagging a target molecule with at least one of said labeled peptides;
• linking the labeled peptide with a linker, said linker providing the linkage to said target molecule;
• linking said labeled peptide or said linker with a spacer, and locating said spacer between linker and target molecule or between said labeled peptide and target molecule, said spacer preventing steric hindrance between said labeled peptide and said target molecule.
